# FASCICULE DE BREVET EUROPEEN

(11) **EP 2 823 067 B1**
(45) Date de publication et mention de la délivrance du brevet: **01.11.2017**
(21) Numéro de dépôt: 13720051.5
(22) Date de dépôt: 04.03.2013
(51) Int. Cl.: C12Q 1/70, C12Q 1/68

(54) **UTILISATION DE RBM39 COMME BIOMARQUEUR**
VERWENDUNG VON RBM39 ALS BIOMARKER
USE OF RBM39 AS A BIOMARKER

(30) Priorité: 05.03.2012 FR 1251980
(43) Date de publication de la demande: 14.01.2015
(73) Titulaire: ABIVAX, 75008 Paris (FR)
(72) Inventeur: TAZI, Jamal, 34380 Clapiers (FR); VENABLES, Julian, Newcastle NE2 3HY (GB); GARCEL, Aude, 34920 Le Cres (FR); CAMPOS, Noëlie, 34920 Le Crès (FR); MAHUTEAU-BETZER, Florence, 78470 Saint-Remy-Les-Chevreuse (FR); NAJMAN, Romain, 94240 L'Hay-Les-Roses (FR); SCHERRER, Didier, 34170 Castelnau Le Lez (FR)
(74) Mandataire: Nony
(86) Numéro de dépôt international: PCT/IB2013/051707
(87) Numéro de publication internationale: WO 2013/132412

(56) Documents cités:
- WO-A2-2010/039778
- WO-A2-2010/143169
- US-A1- 2004 086 500
- JULIAN P VENABLES ET AL: "Cancer-associated regulation of alternative splicing", NATURE STRUCTURAL & MOLECULAR BIOLOGY, vol. 16, no. 6, 1 juin 2009 (2009-06-01), pages 670-676, XP055039474, ISSN: 1545-9993, DOI: 10.1038/nsmb.1608 -& Julian P Venables ET AL: "Cancer-associated regulation of alternative splicing: online supplementary table", Nature structural and molecular biology, 17 mai 2009 (2009-05-17), XP055039721, Extrait de l'Internet: URL:http://www.nature.com/nsmb/journal/v16 /n6/suppinfo/nsmb.1608_S1.html [extrait le 2012-10-02]
- GAUTIER VIRGINIE W ET AL: "In vitro nuclear interactome of the HIV-1 Tat protein", RETROVIROLOGY, BIOMED CENTRAL LTD., LONDON, GB, vol. 6, no. 1, 19 mai 2009 (2009-05-19), page 47, XP021059326, ISSN: 1742-4690, DOI: 10.1186/1742-4690-6-47 -& Virginie W Gautier ET AL: "In vitro nuclear interactome of the HIV-1 Tat protein: online supplementary table", Retrovirology, 19 mai 2009 (2009-05-19), XP055039734, Extrait de l'Internet: URL:http://www.biomedcentral.com/content/s upplementary/1742- 4690-6-47-S1.xls [extrait le 2012-10-02]
- ANTIGONE K MOROU ET AL: "The HIV-1 gp120/V3 modifies the response of uninfected CD4 T cells to antigen presentation: mapping of the specific transcriptional signature", JOURNAL OF TRANSLATIONAL MEDICINE, vol. 9, no. 1, 1 janvier 2011 (2011-01-01) , page 160, XP055039735, ISSN: 1479-5876, DOI: 10.1186/1479-5876-9-160
- ERIC DAILLY ET AL: "Virological response to darunavir in patients infected with HIV is linked to darunavir resistance-associated mutations corrected by the count of mutations with positive impact and is not associated with pharmacological and combined virological/pharmacolog", FUNDAMENTAL & CLINICAL PHARMACOLOGY, vol. 26, no. 4, 5 mai 2011 (2011-05-05), pages 538-542, XP055039738, ISSN: 0767-3981, DOI: 10.1111/j.1472-8206.2011.00949.x
- S. K. KONIG ET AL: "Impact of drug transporters on cellular resistance towards saquinavir and darunavir", JOURNAL OF ANTIMICROBIAL CHEMOTHERAPY, vol. 65, no. 11, 3 septembre 2010 (2010-09-03), pages 2319-2328, XP055039739, ISSN: 0305-7453, DOI: 10.1093/jac/dkq324

### SEQUENCE LISTING

<110> SPLICOS
<120> Utilisation de RBM39 comme biomarqueur
<130> BR97820
<150> FR1251980
   <151> 2012-03-05
<160> 3
<170> BiSSAP 1.0
<210> 1
   <211> 20
   <212> DNA
   <213> Artificial Sequence
<220>
   <221> source
   <222> 1..20
   <223> /mol_type="DNA" /note="amorce" /organism="Artificial Sequence"
<400> 1
   gcaatctctt cccgaacacg 20
<210> 2
   <211> 18
   <212> DNA
   <213> Artificial Sequence
<220>
   <221> source
   <222> 1..18
   <223> /mol_type="DNA" /note="amorce" /organism="Artificial Sequence"
<400> 2
   tcatggccgt tggcactg 18
<210> 3
   <211> 40
   <212> PRT
   <213> Homo sapiens
<220>
   <221> SOURCE
   <222> 1..40
   <223> /mol_type="protein" /organism="Homo sapiens"
<400> 3
   Met Ala Asp Asp Ile Asp Ile Glu Ala Met Leu Glu Ala Pro Tyr Lys
   1               5                   10                  15
   Lys Thr Cys Leu Thr Ile Ser His Leu Tyr Ser His Glu Phe Thr Phe
               20                  25                  30
   Asp Ser Ser Val Asn Cys Gln Ser
           35                  40

## Description

La présente invention concerne un marqueur cellulaire pour le virus de l'immunodéficience humaine (VIH). Plus spécifiquement, la présente invention concerne l'identification d'un marqueur cellulaire utile pour évaluer l'efficacité de composés présumés actifs à l'égard du VIH. Plus particulièrement, la présente invention concerne des procédés de criblage de nouveaux composés actifs à l'égard du VIH ou pour évaluer l'efficacité d'un traitement à l'égard du VIH chez un patient.

Le SIDA (Syndrome d'Immunodéficience Acquise) est une cause importante de mortalité dans le monde, et considéré comme étant une pandémie. L'agent infectieux provoquant le SIDA est le virus de l'immunodéficience humaine (VIH), rétrovirus appartenant à la famille des lentivirus. Ce virus affecte les lymphocytes T, conduisant à une sévère immunodéficience et au développement de différents cancers et infections opportunistes, et, généralement, au décès de l'individu infecté.

L'épissage intracellulaire est un processus consistant à éliminer les introns de l'ARN messager (ARNm) pour produire un ARNm mature, utilisable par les mécanismes de traduction de la cellule (Sharp, Cell, 1994, 77 : 805). Dans le cas de l'épissage alternatif, le même ARNm précurseur peut être, par excision de certains exons, source de différents ARNm codant pour différentes formes d'une protéine, ou isoformes, ayant des fonctions distinctes (Black, Ann Rev Biochem, 2003, 72: 991). Ces évènements sont dénommés évènements d'épissage alternatif ou « alternative splicing event » (ASE). La sélection précise des sites d'épissage en 5' et 3' est un mécanisme qui est source de diversité et qui peut conduire à la régulation de l'expression des gènes selon le type tissulaire ou durant le développement de l'organisme.

Le séquençage du génome humain et l'analyse des banques EST (Expressed Sequence Tag) a montré que 65 % des gènes sont exprimés sous la forme de variants alternativement épissés (Ewin & Green, Nat Genet, 2000, 25 : 232 ; Johnson et al., Science, 2003, 302 : 2141). Il est estimé par ailleurs que 50% des mutations ponctuelles impliquées dans les maladies génétiques induisent un épissage alternatif aberrant.

Ce mécanisme peut donc constituer une cible thérapeutique intéressante, et pas seulement dans le cadre des maladies génétiques.

Ainsi, récemment, de nouveaux composés agissant sur le processus d'épissage alternatif ont été proposés pour le traitement et/ou la prévention d'une infection par VIH (WO 2010/143169).

Le mécanisme cellulaire d'épissage alternatif étant une cible thérapeutique particulièrement intéressante pour le traitement de l'infection par VIH, il existe un besoin de disposer d'un biomarqueur cellulaire permettant d'évaluer l'efficacité d'actifs potentiels à l'égard de ce mécanisme.

Il existe également un besoin de disposer d'un biomarqueur cellulaire utile pour cribler de nouveaux actifs aptes à prévenir et/ou traiter une infection par VIH, et agissant sur l'épissage alternatif

Il existe également un besoin de disposer d'un biomarqueur permettant d'évaluer la réponse d'un individu souffrant d'une infection par VIH au traitement de cette infection.

La présente invention a pour objet de satisfaire à ces besoins.

Ainsi, selon un de ces premiers objets, la présente invention concerne l'utilisation (i) d'un taux d'expression d'une isoforme longue d'une protéine RBM39, dite RBM39L, et (ii) d'un taux d'expression d'une isoforme courte d'une protéine RBM39, dite RBM39C, comme marqueur de l'efficacité d'un actif apte à prévenir et/ou traiter une infection par VIH;
- le rapport entre ledit taux d'expression de l'isoforme longue et ledit taux d'expression de l'isoforme courte étant indicatif de l'efficacité de l'actif à prévenir et/ou traiter la dite infection ;
- la dite isoforme longue correspondant à une isoforme de la protéine RBM39 de référence UniProtKB/Swiss-Prot Q14498-1;
- la dite isoforme courte correspondant à une isoforme de la protéine RBM39 de séquence SEQ ID N°3.

Au sens de l'invention, on entend par « isoforme » une forme particulière d'une protéine obtenue par épissage alternatif. Des protéines isoformes issues de l'épissage alternatif d'un ARNm résultant de la transcription d'un même gène ont des séquences peptidiques proches, mais se différencient les une des autres par l'ajout ou la perte d'une partie de leur séquence peptidique, résultant de l'inclusion ou de l'exclusion d'un exon.

Au sens de l'invention, on entend par « transcrit » un acide ribonucléique (ARN) résultant de la transcription d'un gène. Un gène peut donner plusieurs transcrits ou ARNm par épissage alternatif, chacun codant pour une isoforme, obtenue après traduction de l'ARNm.

Au sens de l'invention, on entend par "expression" d'une isoforme d'une protéine, (i) soit la synthèse d'un ARNm codant ladite isoforme protéique, par transcription du gène correspondant, (ii) soit la synthèse de ladite isoforme protéique par traduction, et le cas échéant modification post-traductionnelle, de ARNm codant ladite isoforme protéique.

Au sens de l'invention, on entend par « marqueur» cellulaire ou « biomarqueur » un constituant d'une cellule ou d'un tissu qui peut être déterminé directement ou indirectement, par exemple par visualisation directe ou après prélèvement et marquage, par exemple avec un marqueur fluorescent ou isotopique, ou encore par une méthode de dosage, et qui est indicatif d'un état physiologique, biochimique ou morphologique de ladite cellule ou dudit tissu. Ainsi, un marqueur cellulaire ou un biomarqueur peut être utilisé pour qualifier un état physiologique, biochimique ou morphologique d'un individu en ayant besoin ou d'un tissu biologique dudit individu.

Au sens de l'invention, on entend par « prévenir », se référer à l'action de réduire le risque de survenue d'un événement.

De manière inattendue, les inventeurs ont observé un déplacement de l'événement d'épissage alternatif (ASE) de l'ARNm issu de la transcription du gène RBM39 vers l'ARNm codant pour l'isoforme longue, ARNmL, par rapport à un ARNm codant pour l'isoforme courte, ARNmC, de la protéine RBM39, lors du traitement de cellules mononucléaires du sang périphérique par des composés actifs à l'égard d'une infection à VIH. Il s'est avéré que le rapport du taux de transcription de l'ARNmL à la somme des taux de transcription des ARNmL et ARNmC issus du gène RBM39 peut être mis en oeuvre comme marqueur sensible et spécifique de l'efficacité d'agents actifs à l'égard d'une infection à VIH.

Les agents actifs considérés dans l'invention sont des agents présumés être actifs à l'égard du VIH et présumés aptes à affecter l'épissage alternatif des gènes, et notamment le ratio des différents ARNm transcrits, et en conséquence le ratio des différentes isoformes protéiques produites.

Selon un autre aspect, la présente invention concerne un procédé de criblage in vitro ou ex vivo d'un actif présumé prévenir et/ou traiter une infection à VIH comprenant au moins les étapes consistant à :
a- mettre en contact :
   i- au moins une cellule convenant à la transcription, par épissage alternatif, d'un ARNm codant pour une isoforme longue, ARNmL, et d'un ARNm codant pour une isoforme courte, ARNmC, d'une protéine RBM39, ladite cellule étant disposée dans des conditions propices à ladite transcription, et
   ii- un actif à cribler,
   dans des conditions propices à l'observation d'un éventuel effet dudit actif sur la
   transcription d'un gène RBM39,
b- déterminer un taux de transcription de ladite isoforme courte et un taux de transcription de ladite isoforme longue dudit gène RBM39,
c- déterminer un rapport entre les taux de transcription déterminés à l'étape b),
d- comparer ledit rapport obtenu à l'étape c) à un rapport de référence;
   - la dite isoforme longue correspondant à une isoforme de la protéine RBM39 de référence UniProtKB/Swiss-Prot Q14498-1;
   - la dite isoforme courte correspondant à une isoforme de la protéine RBM39 de séquence SEQ ID N °3.
Les utilisations et procédés décrits conviennent à une mise en oeuvre *in vitro, ex vivo* ou *in vivo,* et en particulier *in vitro* ou *ex vivo.*

Selon encore un autre de ces objets, la présente invention concerne un procédé d'évaluation de l'efficacité d'un actif destiné à prévenir et/ou traiter une infection à VIH chez un individu en ayant besoin comprenant au moins les étapes consistant à :
a- déterminer, dans un échantillon biologique isolé dudit individu, avant traitement avec ledit actif, un taux d'expression d'une isoforme longue d'une protéine RBM39, RBM39L, et un taux d'expression d'une isoforme courte d'une protéine RBM39, RBM39C,
b- déterminer un rapport entre les taux d'expression déterminés à l'étape a),
c- déterminer, dans un échantillon biologique isolé dudit individu, après traitement avec ledit actif, un taux d'expression d'une isoforme longue d'une protéine RBM39, RBM39L, et un taux d'expression d'une isoforme courte d'une protéine RBM39, RBM39C,
d- déterminer un rapport entre les taux d'expression déterminés à l'étape c), et
e- comparer ledit rapport obtenu à l'étape d) à ledit rapport obtenu à l'étape b);
   - la dite isoforme longue correspondant à une isoforme de la protéine RBM39 de référence UniProtKB/Swiss-Prot Q14498-1;
   - la dite isoforme courte correspondant à une isoforme de la protéine RBM39 de séquence SEQ ID N °3.
Selon certains modes de réalisation du procédé ci-dessus, lesdits taux d'expression consistent respectivement en (i) un taux de transcription d'un ARNm codant pour une isoforme longue, ARNmL, et (ii) un taux de transcription d'un ARNm codant pour une isoforme courte, ARNmC, d'une protéine RBM39.

Selon certains autres modes de réalisation du procédé ci-dessus, lesdits taux d'expression consistent respectivement en (i) un taux de production d'une isoforme longue, ProtL, et en (ii) un taux de production d'une isoforme courte, ProtC, d'une protéine RBM39.

Au sens de l'invention on entend par « échantillon biologique isolé », se référer à tout échantillon biologique obtenu, et isolé, d'un individu, et présumé convenir à la détermination d'un taux de transcription des isoformes longue et courte de la protéine RBM39. Par exemple, un échantillon biologique isolé convenant à l'invention peut être un prélèvement sanguin et peut comprendre des cellules mononucléaires.

Encore selon un autre de ces objets, la présente invention concerne une utilisation d'un kit telle que définie par la revendication 14, ledit kit comprenant au moins une paire d'amorces d'acides nucléiques convenant à la détermination d'un taux de transcription d'un ARNm codant pour une isoforme longue, ARNmL, et un taux de transcription d'un ARNm codant pour une isoforme courte, ARNmC, d'une protéine.

La présente invention a pour avantage de proposer un nouveau marqueur cellulaire sensible, spécifique, et reproductible pour qualifier l'efficacité d'agents actifs à l'égard du VIH et agissant sur l'épissage alternatif.

La présente invention a également pour avantage de proposer des méthodes sensibles, rapides et simples de détermination de l'efficacité d'agents actifs à l'égard d'une infection à VIH.

### Protéine RBM39

La protéine RBM39 (ou RNA-binding protein 39) est présente dans le noyau où elle est co-localisée avec des protéines du noyau splicéosomal. C'est une protéine liant l'ARN qui fonctionne à la fois comme un facteur d'épissage et comme un facteur de régulation de la transcription des gènes (Dowhan et al., Mol Cell, 2005, 17 :429). Des études de la protéine paralogue chez la souris suggèrent que cette protéine peut agir comme un co-activateur de la transcription pour JUN/AP-1 et les récepteurs des oestrogènes (Jung et al., J Biol Chem, 2002, 277 :1229). RBM39 est également impliqué comme co-facteur oncogénique (Dutta et al., J Virol, 2008, 82 :10792) et est impliqué dans l'épissage alternatif du facteur d'angiogenèse VEGF (Dowhan *et al.,* 2005). Enfin, RBM39 a récemment été identifié comme facteur interagissant avec la protéine tat du VIH au moyen d'une stratégie de protéomique basée sur une chromatographie d'affinité couplée à un spectromètre de masse (Gautier et al., Retrovirology, 2009, 6 : 47).

On précise que le gène RBM39 est constitué de l'acide nucléique allant du nucléotide en position 34330259 jusqu'au nucléotide en position 34291128 du brin "moins" du chromosome 20 humain tel que référencé dans la base de données Genbank.

On connait une pluralité d'isoformes de la protéine RBM39. La pluralité d'isoformes de la protéine RBM39 résulte de l'existence d'une grande variété de produits de transcription du gène *RBM39,* du fait d'évènements d'épissage alternatifs. On recense aujourd'hui environ 57 ARNm distincts et environ 34 isoformes de la protéine RBM 39.

Sont documentées ci-dessous exclusivement (i) l'isoforme longue et (ii) l'isoforme courte dont les taux d'expression sont utilisés comme marqueur pour la présente invention.

Une première isoforme, dite "isoforme longue" aux fins de la présente description, ou isoforme 1 ou HCC1.4 (référence UniProtKB/Swiss-Prot Q14498-1) comprend 530 acides aminés et possède un poids moléculaire d'environ 59 380 Da.

Ladite isoforme longue est codée par un ARNm, dit ARNmL, dont la sequence en nucleotides est connue (références Genbank "transcript_id: NM_184234.2", "db_xref: GI:336176061"; "db_xref: GeneID:9584"; "db_xref: HGNC:15923"; "db_xref: HPRD:09201 "; "db_xref: MIM:604739").

Une seconde isoforme, dite "isoforme courte" aux fins de la présente description, comprend 40 acides aminés et possède un poids moléculaire d'environ 4500 Da. Ladite isoforme courte est représentée par la séquence d'acides aminés SEQ ID N° 3.

L'isoforme courte résulte d'un épissage alternatif provoquant l'introduction d'un exon additionnel, entre l'exon n°2 et l'exon n°3 présents sur l'ARNm codant l'isoforme longue. L'exon additionnel (l'exon "n°3" présent sur l'ARNm codant l'isoforme courte) provoque (i) un changement du cadre de lecture et (ii) l'introduction d'un codon stop.

Ladite isoforme courte est codée par un ARNm, dit ARNmC. Plus précisément, l'isoforme courte de séquence SEQ ID N°3 est codée par un pluralité d'ARN messagers qui résultent tous d'un évènement d'insertion dudit exon additionnel provoquant un changement du cadre de lecture et l'introduction dudit codon stop. Les différents ARN messagers codant l'isoforme courte de RBM39 de séquence SEQ ID N°3 sont collectivement désignés "ARNmC" dans la présente description.

La séquence d'acides aminés allant du résidu en position 1 jusqu'au résidu en position 17 de la séquence de l'isoforme courte de SEQ ID N° 3 est identique à la séquence d'acides aminés allant du résidu en position 1 jusqu'au résidu en position 17 de la séquence de 530 aminés de l'isoforme longue (référence UniProtKB/Swiss-Prot Q14498-1). En revanche, du fait du changement du cadre de lecture généré par la présence de l'exon additionnel dans ARNmC, la séquence allant du résidu 18 jusqu'au résidu 40 de l'isoforme courte de séquence SEQ ID N°3 diffère de la séquence correspondante de ladite isoforme longue.

Il est montré selon l'invention que, lorsque le gène RBM 39 est transcrit de manière physiologique dans les cellules du système immunitaire, les ARNm (collectivement ARNmC) codant pour l'isoforme coute sont transcrits de manière prédominante par rapport à l'ARNm codant pour l'isoforme longue.

On précise qu'il n'y a pas spécifiquement de relation entre (i) la longueur d'une isoforme de la protéine RBM39 et (ii) la longueur de l'ARNm codant ladite isoforme. A titre illustratif, ARNmL qui code l'isoforme longue de RBM39 est plus court que les ARNmC qui codent l'isoforme courte de RBM39. La longueur accrue de ARNmC, par rapport à l'ARNmL, résulte de la présence de l'exon additionnel générant un codon stop précoce dans l'ARNmC.

On précise que ARNmL peut être aisément discriminé des ARN messagers codant l'isoforme courte (collectivement ARNmC) du fait de la longueur plus courte de ARNmL, par rapport à la longueur de chacun des ARN messagers codant l'isoforme courte.

Ainsi, un déplacement de l'équilibre de transcription du gène codant RBM39 vers une réduction du taux de transcription de l'ARNmL comparé au taux de transcription de l'ARNmC peut indiquer une modification des mécanismes d'épissage alternatif affectant la transcription de ce gène, et plus globalement de la physiologie de la cellule et des infections susceptibles de l'affecter.

On précise qu'un déplacement de l'équilibre de transcription du gène codant RBM39 vers une réduction du taux de transcription de l'ARNmL comparé au taux de transcription de l'ARNmC induit parallèlement un déplacement de l'équilibre de la synthèse ou production de la protéine RBM39 vers une réduction du taux de production de l'isoforme longue ProtL comparé au taux de production de l'isoforme courte ProtC.

### Utilisations et Procédés

Les utilisations et procédés de l'invention peuvent, en particulier, convenir à qualifier ou à au cribler un agent présumé actif à l'égard d'une infection à VIH, ou peuvent convenir à la détermination de l'efficacité d'un traitement d'un individu ayant une infection à VIH au moyen de ces actifs.

Un aspect de l'invention comprend la détermination des taux d'expression des isoformes courtes (RBM39C) et longue (RBM39L) d'une protéine RBM39.

Cet aspect comprend la détermination des taux de transcription des ARNm codant pour les isoformes courte (ARNmC) et longue (ARNmL) d'une protéine RBM39.

Cet aspect de l'invention comprend aussi la détermination du taux de production des isoformes longue, dite ProtL, et courte, dite ProtC, d'une protéine RBM39

Le taux de transcription des ARNmC et ARNmL peut être déterminé en mesurant la quantité d'ARNm correspondant directement produit par la cellule, ou peut être déterminé sur de l'ADN obtenu par transcription inverse de l'ARNm au moyen d'une méthode permettant de conserver la proportion des différents ARNm.

Le taux de production des ProtC et ProtL peut être déterminé en mesurant la quantité de protéine correspondante produite par la cellule, par exemple à l'aide de méthodes mettant en oeuvre (i) soit des moyens détectables reconnaissant spécifiquement à la fois les deux isoformes ProtC et ProtL d'une protéine RBM39, (ii) soit des moyens détectables reconnaissant spécifiquement une seule des deux isoformes ProtC et ProtL d'une protéine RBM39, lesquels peuvent être utilisés en combinaison.

Selon un aspect de l'invention, la détermination des taux de transcription des ARNm peut se faire sur l'ARNm entier ou sur une portion caractéristique de l'ARNm. Une portion caractéristique est une portion incluant tout ou partie du ou des exons faisant l'objet de l'épissage alternatif.

Avantageusement, la détermination des taux de transcription peut être effectuée sur une portion caractéristique des ARNmC et ARNmL issus de la transcription du gène RBM39 au moyen d'un procédé comprenant une étape d'amplification spécifique de cette portion.

Les méthodes de détection et de mesure de quantité de l'ARN ou de l'ADN sont connues de l'homme de l'art. Ces méthodes englobent les méthodes de RT-PCT (pour "Reverse transcriptase PCR") et de qRT-PCR (pour "quantitative Reverse Transcriptase PCR") et de Real-Time PCR. Pour la mise en oeuvre de ces méthodes, l'homme du métier peut se référer notamment aux travaux de Wang et al. (1989, Proc Natl Acad Sci USA, Vol. 86 : 917-921), de Wong et al. (2005, Bio Techniques, Vol. 39 (1): 75-85), de Nolan et al. (2006, Nat Protoc, Vol. 1(3) : 1559-1582) et de Klinck et al. (2008, Cancer Research, Vol. 68 : 657-663), ou encore à une revue générale de ces techniques publiées par Bustin (2000, Journal of Molecular Endocrinology, Vol. 25 : 169-193).

L'ensemble de ces méthodes comprennent (i) une étape d'extraction des ARNm cellulaires, (iii) une étape de transcription inverse de ARNm en ADN à l'aide d'une enzyme transcriptase inverse et (iii) une étape d'amplification de l'ADN obtenu à l'étape précédente, en utilisant des amorces nucléotidiques appropriées, avant quantification de l'ADN amplifié. En général, sont simultanément amplifiés à partir du même échantillon (a) l'ADN obtenu par transcription inverse de l'ARNm d'intérêt et (b) un ADN ou une pluralité d'ADNs obtenus par transcription inverse d'ARNm exprimés de manière constitutive et constante par les cellules ("housekeeping genes"), tels que les ARNs codés par les gènes *MRPL19, PUM1* et *GADPH.*

L'ADN amplifié peut être quantifié, après séparation par électrophorèse et, par mesure des bandes d'ADN, et les résultats pour le ou les ARNm d'intérêt exprimés en valeurs relatives par comparaison avec les ARNm codés par les gènes "housekeeping". Dans certains modes de réalisation, l'étape de séparation des ADNs amplifiés est réalisée par électrophorèse sur gel d'agarose, puis coloration des bandes d'ADN au bromure d'éthidium, avant quantification des ADNs contenu dans les bandes de migration par densitométrie. Dans d'autres modes de réalisation, on utilise un dispositif à micro-canaux dans lequel est réalisée une séparation des ADNs amplifiés par électrophorèse capillaire, puis une quantification des différents ADNs séparés par mesure du signal émis par ces ADNs après illumination par un faisceau laser. Un tel dispositif peut être l'appareil LabChip®, par exemple de la série"GX", commercialisé par la Société Caliper LifeSciences (Hopkinton, MA, Etats-Unis).

Dans certains modes de réalisation, la détermination des taux de transcription des ARNm peut comprendre une étape d'amplification conjointe des ADNc résultant de la transcription inverse des ARNmL et ARNmC à l'aide d'une paire unique d'amorces nucléotidiques, les deux types d'ADNc amplifiés résultants étant ensuite séparés, préférentiellement par électrophorèse.

Dans d'autres modes de réalisation, la détermination des taux de transcription des ARNm peut comprendre une étape d'amplification d'une portion caractéristique des ARNmL et ARNmC issus de la transcription du gène RBM39, à l'aide de deux paires d'amorces distinctes, respectivement pour les ADNc obtenu à partir de chacun des ARNmL et ARNmC.

Différentes amorces sens et anti-sens peuvent être mises en oeuvre dans une réaction de RT-PCR convenant à l'invention, sous réserve qu'elles encadrent l'exon faisant l'objet de l'inclusion ou de l'exclusion. Le choix des amorces sens et anti-sens peut également être guidé par la différence de longueurs entre les transcrits issus des ARNmL et ARNmC à amplifier. La différence de longueur peut être ajustée selon la méthode de séparation des transcrits amplifiés et du degré de résolution qu'elle permet d'obtenir.

Avantageusement, des amorces convenant à l'invention peuvent être choisies pour amplifier une portion d'ARNm de RBM 39 susceptible d'inclure l'exon additionnel, et comprenant de chaque côté de cet exon au moins 18 nucléotides.

Avantageusement, la différence de taille entre les transcrits issus des ARNmL et ARNmC issus de la transcription du gène RBM 39 est de 73 nucléotides.

Dans des modes de réalisation avantageux, un couple d'amorces est choisi de telle sorte que :
- une première amorce hybride spécifiquement avec la région d'un ARNm issu de la transcription du gène RBM39 correspondant à l'exon n°1 (exon référencé n°1 dans l'ARNmL codant l'isoforme longue). L'exon n° 1 consiste en l'acide nucléique allant du nucléotide en position 5001 jusqu'au nucléotide en position 5396 de la séquence du gène RBM39 (référence Genbank n° NG_029955), et
- une seconde amorce hybride spécifiquement avec la région d'un ARNm issu de la transcription du gène RBM39 correspondant à l'exon n°3 (exon référencé n°3 dans l'ARNmL codant l'isoforme longue). L'exon n° 3 consiste en l'acide nucléique allant du nucléotide en position en position 6740 jusqu'au nucléotide en position 6812 de la séquence du gène RBM39 (référence Genbank n° NG_029955).

Des amorces sens et anti-sens convenant à l'invention peuvent être obtenus par tout procédé connu de l'homme de l'art dans le domaine, notamment comme décrit par Sambrook et al. (Molecular Cloning: A Laboratory Manual, 3rd ED., 2001, Cold Spring Harbour, N. Y.).

Dans certains modes de réalisation, on utilise une paire d'amorces nucléotidiques comprenant respectivement les séquences SEQ ID N°1 et SEQ ID N°2. L'amorce nucléotidique de séquence SEQ ID N° 1 hybride avec une région d'un ARNm issu de la trasncription du gène RBM39, qui comprend l'exon 3 dudit gène (hybride avec la séquence complémentaire de l'acide nucléique comprenant les nucléotides 34329893 à 34329912 du brin "moins" du chromosome 20 selon Genbank). L'amorce nucléotidique de séquence SEQ ID N° 2 hybride avec une région d'un ARNm issu de la trasncription du gène RBM39, qui comprend l'exon 1 dudit gène (hybride avec la séquence complémentaire de l'acide nucléique comprenant les nucléotides 34326905 à 34326922 du brin "moins" du chromosome 20 selon Genbank).

Selon un aspect de l'invention, les déterminations des taux de transcription des ARNmL et ARNmC issus de la transcription du gène RBM39 peuvent être mis en oeuvre sous forme d'un rapport.

Avantageusement, ce rapport peut être déterminé en établissant un rapport entre un taux de transcription de l'ARNm codant pour une isoforme longue d'une protéine RBM39 et un taux de transcription de l'ARNm codant pour une isoforme courte d'une protéine RBM39.

De manière plus avantageuse encore, on peut établir un rapport entre un taux de transcription de l'ARNm codant pour une isoforme longue d'une protéine RBM39 et la somme des taux de transcription des ARNm codant pour les isoformes longue et courte d'une protéine RBM39. Ce rapport permet de déterminer une valeur en pourcentage d'épissé (Ψ).

Selon un autre aspect de l'invention, un rapport ainsi déterminé peut être comparé à un rapport de référence.

Au sens de l'invention, on entend par « rapport de référence » se référer à un rapport déterminé au moyen de taux de transcription d'ARNm mesurés dans des conditions de base ou qualifié de physiologiquement normale.

Par exemple, de tels taux de transcription peuvent être déterminés dans des cellules disposées dans des conditions physiologiques ou des conditions mimant des conditions physiologiques, et en l'absence de facteurs externes susceptibles d'affecter ces conditions. Un facteur externe considéré dans l'invention peut être un agent actif présumé efficace pour prévenir et/ou traiter une infection à VIH.

Un rapport de référence peut être obtenu parallèlement au rapport déterminé en présence de facteur externe susceptible d'affecter la transcription des ARNm d'intérêts, ou peut être déterminé antérieurement voire postérieurement.

Un rapport de référence peut être stocké sur un support, par exemple électronique, pour constituer une base de données dite de référence.

Selon un aspect de l'invention, lors de la mise en oeuvre de l'invention pour évaluer l'efficacité d'un actif apte à prévenir et/ou traiter une infection à VIH, un rapport de référence peut être obtenu par détermination du taux de transcription des ARNm d'intérêt en l'absence de l'actif dont l'efficacité est à évaluer.

En particulier, lors de la mise en oeuvre de l'invention pour cribler un actif présumé prévenir et/ou traiter une infection à VIH, un rapport de référence peut être déterminé en reproduisant le protocole de criblage en l'absence de l'actif à cribler.

En particulier, lors de la mise en oeuvre de l'invention pour évaluer l'efficacité d'un actif destiné à prévenir et/ou traiter une infection à VIH chez un individu en ayant besoin un rapport de référence peut être déterminé dans un échantillon biologique isolé obtenu dudit individu avant l'administration de l'actif.

Alternativement, un rapport de référence peut être déterminé chez un ensemble d'individus non soumis au traitement dont l'efficacité est à évaluer.

Lors de la mise en oeuvre de l'invention pour évaluer l'efficacité d'un actif destiné à prévenir et/ou traiter une infection à VIH chez un individu en ayant besoin, le rapport de référence peut, le cas échéant, être corrélé à d'autres paramètres du patient, tel que son âge, son poids ou d'autres paramètre physio-pathologiques.

Une déviation entre un rapport déterminé en présence d'un facteur susceptible d'affecter le taux de transcription des ARNm d'intérêts, tel qu'un agent actif, et un rapport de référence peut être indicative d'un éventuel effet dudit facteur, tel que ledit agent actif.

Une telle déviation peut être indicative d'un déplacement de l'événement d'épissage alternatif du taux de transcription de l'ARNmL vers le taux d'ARNmC, des ARNm issus de la transcription du gène RBM39. Un tel déplacement peut être indicatif d'un éventuel effet du facteur étudié, tel que l'efficacité d'un agent présumé actif à l'égard d'une infection à VIH et agissant sur les mécanismes d'épissage alternatifs.

Une telle déviation peut résulter d'une diminution de la valeur du rapport entre un taux de transcription de l'ARNm codant pour une isoforme longue d'une protéine RBM39 et la somme des taux de transcription des ARNm codant pour les isoformes longue et courte d'une protéine RBM39.

Selon un aspect de l'invention, une déviation entre un rapport des taux de transcription des ARNmL et ARNmC codant pour une protéine RBM39 déterminé en présence d'un agent présumé actif à l'égard du VIH et un rapport de référence peut être indicative d'un éventuel effet dudit agent actif à l'égard du VIH.

Selon un autre aspect de l'invention, une déviation entre un rapport des taux de transcription des ARNmL et ARNmC codant pour une protéine RBM39 déterminé chez un individu traité avec un agent présumé actif à l'égard du VIH et un rapport de référence peut être indicative d'un éventuel effet dudit agent actif à l'égard du VIH ou d'une sensibilité de l'individu au traitement.

Selon le degré de déviation observé, un individu traité peut être qualifié de résistant, de faiblement résistant, de faiblement sensible, ou de sensible au traitement.

Ainsi, une mise en oeuvre de l'invention peut également permettre de déterminer la résistance, voir le degré de résistance, ou la sensibilité ou le degré de sensibilité du VIH infectant un individu au traitement destiné à être administré audit individu.

Selon un autre aspect, l'invention peut être mise en oeuvre pour suivre la progression de la résistance du VIH au traitement anti-VIH chez un individu présentant une infection à VIH.

Un tel mode de réalisation peut comprendre l'obtention d'un premier échantillon biologique isolé d'un individu présentant une infection à VIH et soumis à un traitement anti-VIH à un temps t₀, la détermination d'un rapport des taux de transcription des ARNmL et ARNmC codant pour une protéine RBM39, et la comparaison du rapport obtenu à un rapport des taux de transcription des ARNmL et ARNmC codant pour une protéine RBM39 déterminé dans un second échantillon biologique isolé dudit individu à un temps ultérieur t₁.

L'observation d'une déviation ou d'une absence de déviation entre les deux rapports déterminés peut être indicative avec la progression ou l'absence de progression de l'infection à VIH au traitement anti-VIH. L'obtention d'échantillons biologiques isolés additionnels dudit individu et la détermination des rapports des taux de transcription des ARNmL et ARNmC codant pour une protéine RBM39 peut être effectué autant de fois que désiré. La possibilité de suivre un individu en effectuant une série de déterminations de rapports de taux de transcription des ARNmL et ARNmC codant pour une protéine RBM39 au cours du temps peut être utilisé pour suivre la progression de la résistance d'une infection à VIH au traitement administré.

Selon une autre variante préférée de réalisation, un procédé de l'invention peut mettre en oeuvre une cellule choisie parmi les cellules mononuclées du sang périphérique, des cellules HeLa, Jurkat, CEM ou toutes autre cellules susceptibles d'être infectée par le virus du SIDA.

Selon une autre variante préférée de réalisation, dans un procédé de l'invention les taux de transcription des isoformes courte et longue du gène RBM39 peuvent être obtenus au moyen d'une méthode choisie parmi une RT-PCR, RTqPCR.

Selon d'autres modes de réalisation d'un procédé de l'invention, les taux d'expression de l'isoforme longue RBM39L et de l'isoforme courte RBM39C de la protéine RBM 39 consistent respectivement en (i) un taux de production d'une isoforme longue, dite ProtL, et en (ii) un taux de production d'une isoforme courte, dite ProtC, d'une protéine RBM39.

Dans ces modes de réalisation, lesdits taux d'expression sont déterminés par des méthodes comprenant une étape de quantification des ProtL et ProtC produites par les cellules.

Typiquement, les isoformes ProtL et ProtC peuvent être détectés et quantifiés à l'aide de composés ligands reconnaissant spécifiquement (i) soit indifféremment les deux isoformes ProtL et ProtC, (ii) soit spécifiquement une seule des deux isoformes ProtC et ProtL, auquel cas deux composés ligands spécifiques respectivement de l'isoforme ProtL et de l'isoforme ProtC sont nécessaires.

Les composés ligands des isoformes ProtC et ProtL peuvent être choisis parmi les anticorps, les aptamères nucléiques et les aptamères protéiques, dont les méthodes d'obtention sont bien connues de l'homme du métier.

En particulier, des aptamères nucléiques se liant spécifiquement à l'isoforme ProtL, le cas échéant à l'isoforme ProtC, ou encore aux deux isoformes ProtL et ProtC, peuvent être obtenues selon la technique SELEX décrite par exemple dans les brevets américains n° US 5,475,096 et n° US 5,270,163.

Des anticorps dirigés spécifiquement contre chacune des isoformes ProtL et ProtC, et des anticorps reconnaissant spécifiquement à la fois les deux isoformes ProtC et ProtL, peuvent être également préparés selon une grande variété de techniques connues de l'homme du métier. De tels anticorps englobent les anticorps polyclonaux et les anticorps monoclonaux, y compris les anticorps obtenus par recombinaison génétique. Les anticorps peuvent être obtenus suivant l'enseignement de Kohler et Milstein (1975, Nature, Vol. 256 : 495-497). Ils peuvent être obtenus selon des techniques de clonage génétique à partir d'une cellule lymphocytaire unique d'un animal immunisé de manière approprié, comme décrit par Babcook et al. (1996, Proc Natl Acad Sci USA, Vol. 93(15) : 7843-78841) ou encore dans les demande PCT n° WO 92/02551, WO 2004/051268 et WO 2004/106377. Ils peuvent être des anticorps recombinants, en particulier des anticorps humanisés, et peuvent être obtenus par les techniques décrites dans le brevet américain n° US 5,585,089 ou dans la demande PCT n° WO 91/09967, ou bien encore dans les documents de brevet EP0546073, US 5,545,806, US 5,569,825, US 5,625,126, US 5,633,425, US 5,661,016, US5,770,429, EP 0438474 et EP0463151.

De nombreux anticorps reconnaissant spécifiquement la protéine RBM39, et en particulier des régions spécifiques de la protéine RBM39 sont disponibles dans le commerce. On peut utiliser par exemple l'un quelconque des anticorps anti-RBM39 humaine commercialisé par la Société Sigma Aldrich, et notamment les anticorps référencés respectivement #SAB2101959, #SAB2103105 et #HPA001591. On peut aussi utiliser désigné "Caper (P14) commercialisé par la Société Santa Cruz Biotechnology Inc.

Sont également disponibles dans le commerce des dispositifs de test ELISA pour la détection ou la quantification de la protéine RBM39.

Dans certains modes de réalisation d'un procédé selon l'invention, on utilise un anticorps reconnaissant spécifiquement la région C-terminale de la protéine RBM39 (isoforme ProtL). Comme anticorps reconnaissant la partie C-terminale de RBM39, on peut utiliser par exemple l'anticorps #A300-353A qui reconnaît l'épitope compris dans la région 480-530, commercialisé par la Société Bethyl Laboratories (Montgomeryn TXn USA). Alternativement, on peut utiliser l'anticorps #IHC-00022 également commercialisé par la Société Bethyl Laboratories, qui reconnait la région 325-375 de la protéine RBM39.

Les isoformes ProtL et ProtC peuvent être détectées et quantifiées selon des techniques bien connues de l'homme du métier, comme par exemple selon une technique d'immunoempreinte (aussi désignée "Western Blotting"). Typiquement, les protéines cellulaires sont soumises après extraction à une étape de migration sur gel d'électrophorèse, puis les bandes protéiques sont incubées avec l'anticorps anti-RBM39, ou bien avec une combinaison d'anticorps reconnaissant respectivement ProtL et ProtC. Les isoformes ProtL et ProtC peuvent être distinguées selon leur position de migration sur le gel d'électrophorèse. Puis, les isoformes ProtL et ProtC sont quantifiées, par exemple par mesure d'un signal détectable émis par les anticorps utilisés, par exemple par mesure de fluorescence si les anticorps anti-RBM39, ou bien des seconds anticorps anti-anticorps si des seconds anticorps sont utilisés, sont marqué à l'aide d'un chromophore ou un fluorophore, ou bien par mesure d'absorbance si lesdits anticorps sont couplés à une enzyme capable de catalyser la formation d'un produit absorbant la lumière à une longueur d'onde donnée.

La présente invention est relative à un procédé de criblage in vitro ou ex vivo d'un actif présumé prévenir et/ou traiter une infection à VIH comprenant au moins les étapes consistant à :
a- mettre en contact :
   i- au moins une cellule convenant à la production, par épissage alternatif de l'ARNm, d'une isoforme longue de la protéine RBM39, ProtL, et d'une isoforme courte de la protéine RBM39, ProtC, ladite cellule étant disposée dans des conditions propices à ladite production, et
   ii- un actif à cribler,
   dans des conditions propices à l'observation d'un éventuel effet dudit actif sur la transcription d'un gène RBM39,
b- déterminer un taux de production de ladite isoforme courte et un taux de production de ladite isoforme longue de ladite protéine RBM39,
c- déterminer un rapport entre les taux de production déterminés à l'étape b),
d- comparer ledit rapport obtenu à l'étape c) à un rapport de référence.

Au sens de l'invention, on entend par "rapport de référence", dans le contexte du procédé ci-dessus, se référer à un rapport déterminé au moyen de taux de production de protéines mesurés dans des conditions de base ou qualifié de physiologiquement normale.

Avantageusement, ce rapport peut être déterminé en établissant un rapport entre un taux de production de l'isoforme longue d'une protéine RBM39 et un taux de production de l'isoforme courte d'une protéine RBM39.

De manière plus avantageuse encore, on peut établir un rapport entre un taux de production de l'isoforme longue d'une protéine RBM39 et la somme des taux de production des isoformes longue et courte d'une protéine RBM39. Ce rapport permet de déterminer une valeur en pourcentage d'épissé (Ψ).

Les détails de mise en oeuvre des procédés ci-dessus avec les valeurs de taux de production des isoformes ProtL et ProtC sont aisément déterminées par l'homme du métier, au vu de l'enseignement pour la mise en oeuvre des procédés pour lesquels les taux d'expression des isoformes de la protéine RBM39 consistent en des taux de transcription d'un ARNm codant pour lesdites isoformes, qui est divulgué dans la présente description.

La présente invention concerne aussi un procédé d'évaluation de l'efficacité d'un actif destiné à prévenir et/ou traiter une infection à VIH chez un individu en ayant besoin comprenant au moins les étapes consistant à :
a- déterminer, dans un échantillon biologique isolé dudit individu, avant traitement avec ledit actif, un taux de production d'une isoforme longue d'une protéine RBM39, ProtL, et
un taux d'expression d'une isoforme courte d'une protéine RBM39, ProtC,
b- déterminer un rapport entre les taux de production déterminés à l'étape a),
c- déterminer, dans un échantillon biologique isolé dudit individu, après traitement avec ledit actif, un taux de production d'une isoforme longue d'une protéine RBM39, ProtL, et un taux de production d'une isoforme courte d'une protéine RBM39, ProtC,
d- déterminer un rapport entre les taux de production déterminés à l'étape c), et
e- comparer ledit rapport obtenu à l'étape d) à ledit rapport obtenu à l'étape b).

### Kits

Un kit convenant à une utilisation de l'invention peut comprendre au moins une paire d'amorces convenant à la détermination d'un taux de transcription d'un ARNm codant pour une isoforme longue, ARNmL, et d'un taux de transcription d'un ARNm codant pour une isoforme courte, ARNmC, d'une protéine RBM39.

Les amorces sens et anti-sens peuvent être conditionnés dans deux récipients distincts ou peuvent être conditionnés dans un même récipient. Outre ces amorces, un kit peut comprendre l'ensemble des enzymes et réactifs nécessaires à la mise en oeuvre des méthodes de détection des transcrits d'un gène, notamment tel que détaillé précédemment.

Un kit convenant à une utilisation de l'invention peut en outre comprendre une notice explicative précisant les modalités de détermination d'un rapport des taux de transcription d'un ARNm codant pour une isoforme longue, ARNmL, et d'un ARNm codant pour une isoforme courte, ARNmC, d'une protéine RBM39 et de comparaison du rapport obtenu à un rapport de référence.

Dans d'autres modes de réalisation, un kit convenant à une utilisation de l'invention peut comprendre au moins un anticorps reconnaissant à la fois l'isoforme longue ProtL et l'isoforme courte ProtC de la protéine RBM39, ou alternativement au moins deux anticorps, respectivement un anticorps reconnaissant spécifiquement l'isoforme longue ProtL de la protéine RBM39 et un anticorps reconnaissant l'isoforme courte ProtC de la protéine RBM39.

Le cas échéant, le ou les anticorps anti-RBM39 sont marqués par une molécule détectable.

Le cas échéant, ledit kit comprend aussi des anticorps, dits seconds anticorps, reconnaissant les anticorps anti-RBM39, par exemple des anticorps reconnaissant la partie Fc des anticorps anti-RBM39, lesdits seconds anticorps étant marqués par une molécule détectable, par exemple une molécule chromophore, une molécule fluorophore, ou encore ne enzyme apte à catalyser la transformation d'un substrat chromogène.

### LEGENDE DES FIGURES

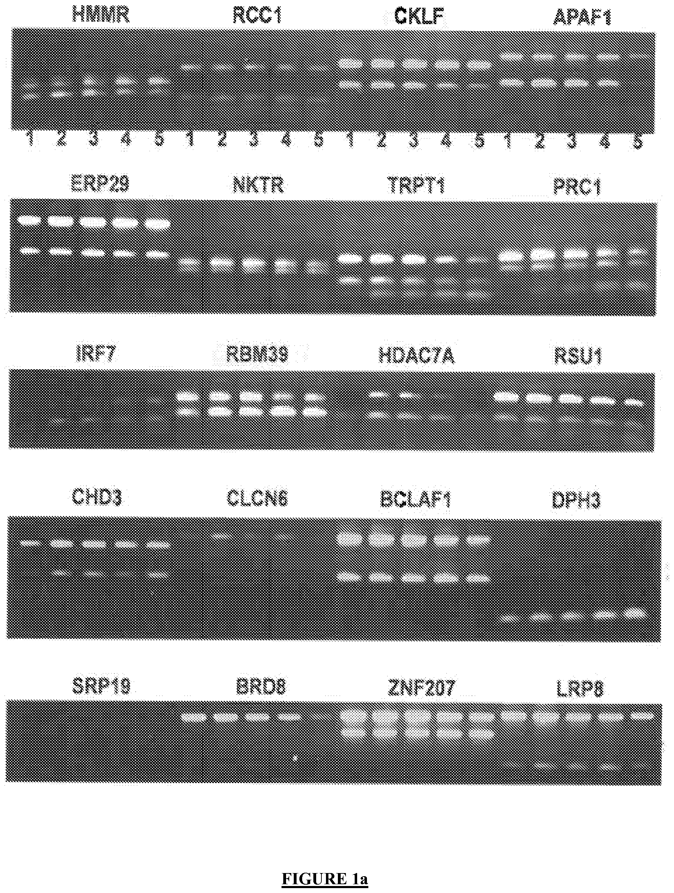

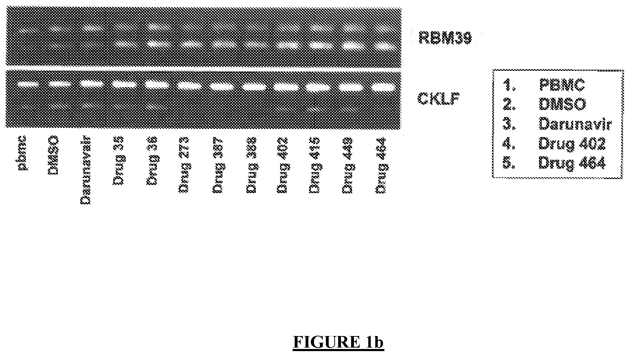

**Figure 1**: La Figure 1A illustre les résultats obtenus par RT-PCR et électrophorèse sur gel d'agarose avec les 20 touches chacune non-spécifiquement associée à un composé et identifiées à l'exemple 1, dans cinq conditions : (1) PBMC, (2) PBMC + DMSO, (3) Darunavair, (4) drug402, et (5) drug464. Les isoformes longues et courtes sont visibles pour la plupart des ASE mais un déplacement significatif de l'épissage vers l'une ou l'autre des isoformes n'a pu être observé que pour les gènes CKLF et RBM39.
La Figure 1B illustre une nouvelle détermination des ASE pour les gènes CKLF et RBM39 dans les mêmes conditions que celles utilisées à l'exemple 1, et seul l'épissage alternatif du gène RBM39 a été modifié de manière constante par les composés testés. La transcription de l'isoforme courte est induite par les composés testés au détriment de l'expression de l'isoforme longue.

Les différents aspects de la présente invention sont illustrés par les exemples détaillés ci-après, qui ne doivent pas être interprétés, de quelque manière que ce soit, comme limitant la portée de l'invention.

### EXEMPLES

### Exemple 1 : Identification des taux d'épissage alternatifs de RBM39 comme marqueur de l'efficacité d'un actif utile dans le traitement d'une infection par HIV.

### A. Matériels et méthodes

### 1. Méthodologie générale

Les réactions d'épissages alternatifs étudiés ont été choisies suite à des criblages successifs de la base de données RefSeq et en considérant les épissages alternatifs connus pour se manifester de manière sensible et constante dans différentes lignées cellulaires.

Pour la recherche d'un marqueur candidat, 382 événements d'épissage alternatifs (Alternative Splice Event, ASE) ont été pré-sélectionnés et représentent un cliché aléatoire à haut débit des altérations globales d'épissage alternatif. Sur les 382 ASE pré-sélectionnés, seuls 309 ASE ont pu être détectés de manière satisfaisante dans les PBMC.

Les 309 événements d'épissages alternatifs (Alternative Splice Events ou ASE) (axe des y) ont été testés par PCR effectuée sur des régions connues d'épissages alternatifs dans douze échantillons (axe des x) : PBMC (cellules), PBMC + DMSO (DMSO), PBMC + contrôle (Darunavair), PBMC + composés testés (drug387, drug464, drug273, drug388, drug449, drug36, drug35, drug402, drug415). L'exclusion ou l'inclusion d'un exon conduit à deux produits de PCR différents : une isoforme courte et une isoforme longue.

Les composés drug35 et drug36, correspondent respectivement aux composés C24 et C25 de WO 2009/087238 (WO 2009/087238, page 45). Ils sont préparés comme décrit dans ce document.

Les composés drug387, drug464, drug273, drug388, drug449 et drug402 sont décrits dans WO 2010/143169 :
- drug387 est le composé 77, de formule Ib (page 51)
- drug464 est le composé 90, de formule Ib (page 53)
- drug273 est le composé 6, de formule Ia (page 44)
- drug388 est le composé 112, de formule Ie (page 56)
- drug449 est le composé 43, de formule Ia (page 47)
- drug402 est le composé 80, de formule Ib (page 52)
- drug415 est le composé 106, de formule Ic (page 56).

### 2. Technique utilisée

Des cellules mononucléées du sang périphérique (ou PBMC : Peripheral Blood Mononuclear Cells) purifiées par centrifugation sur coussin de Ficoll® à partir de poches de sang (donneur sains) fournies par le Centre de transfusion sanguine de Paris (France).

L'ARN est extrait des cellules par la méthode Tri reagent® (Sigma) et les ADN complémentaires des ARNm (ADNc) codant pour les isoformes longues et courtes de RBM39 sont amplifés par RT (*Reverse Transcriptase*)-PCR afin d'obtenir les deux isoformes de l'épissage ARNmL et ARNmC. On a utilisé le kit commercial *"First strand cDNA synthesis kit*" commercialise par la Société GE HealthCare (Etats-Unis).

Typiquement, les réactions de RT-PCR ont été réalisées avec 60 ng ARNm comme substrat dans un volume final de réaction de 50 µl finale contenant 50 pmoles de chaque amorce, respectivement (i) l'amorce sens : GCAATCTCTTCCCGAACACG (SEQ ID N°1), et (ii) l'amorce antisens TCATGGCCGTTGGCACTG (SEQ ID N°2) et la polymérase Taq platinum® (Société Invitrogen).

Les conditions des réactions d'amplification de l'ADNc par PCR sont les suivantes : (a) 2 min à 94°C puis (b) 35 cycles de 30s avec les séquences suivantes *(b1)* 94°C, *(b2)* 55°C et *(b3)* 72°C, suivis de (c) 2 min d'élongation à 72°C.

Les produits de PCR sont analysés sur gel d'agarose 1.5% et les fragments amplifiés sont visualisés par coloration au bromure d'ethidium.

Pour chaque événement d'épissage alternatif, douze conditions ont été testées : PBMC (cellules), PBMC + DMSO (DMSO), PBMC + contrôle (Darunavair), PBMC + composés testés (respectivement les composés suivants : drug387, drug464, drug273, drug388, drug449, drug36, drug35, drug402, drug415).

Chaque composé est testé après activation des cellules par 100U/ml de l'IL2 et infection par VIH1 comme cela est décrit dans la Demande internationale WO2010/143169 déposée le 14 juin 2010 sous le n° PCT/IB2010/052651 aux noms de Splicos, du Centre National de la recherche Scientifique, de l'Institut Curie et de l'Université de Montpellier 2 et dans la demande internationale WO2010/143170 déposée le 14 juin 2010 sous le n° PCT/IB2010/052652 aux noms de Splicos, du Centre National de la recherche Scientifique, de l'Institut Curie et de l'Université de Montpellier 2.

Les premiers résultats ont concerné les données générées par 3708 réactions de PCR.

Un pourcentage d'épissage en valeur (psi ou Ψ) est calculé pour chaque réaction en déterminant un rapport de la concentration en isoforme longue sur la somme des concentrations des isoformes courtes et longues ou L/(L+S). Les valeurs Ψ obtenues sont alternativement codées en couleur en cartes de température. La couleur rouge indique un déplacement de l'épissage vers l'exclusion d'un exon et la couleur verte indique un déplacement de l'épissage vers l'inclusion d'un exon.

On a calculé les valeurs Ψ à la fois pour les gènes dont l'épissage a été altéré uniquement par un ou deux des composés testés et pour les autres gènes dont l'épissage est altéré par plusieurs ou la totalité des composés testés.

L'exclusion ou l'inclusion d'un exon conduit à deux produits de PCR différents : une isoforme courte et une isoforme longue.

Une analyse PCR à haut débit de ces événements d'épissages alternatifs a été effectuée selon la technique décrite par Klinck et al. (2008, Cancer Res, 2008, 68 :657).

### B. Résultats

Les ASE ont été retenus comme significatifs si plus de 75 % du produit de PCR a migré à la mobilité attendue (qualifiant la pureté de la réaction) et si la concentration du produit total attendu en PCR était supérieure à 20 nM (degré d'intensité de la réaction). 309 ASE satisfaisant à ces conditions de sélection ont été identifiés dans les 12 conditions testées, et sont représentés en carte de température en Figure 1A.

Une « touche » ASE est définie pour tout déplacement de l'ASE de plus de 20 % par rapport à une moyenne déterminée sur 3 valeurs contrôle. 40 « touches » ASE ont ainsi été définies : 20 « touches » ASE correspondant à un déplacement sporadique induit uniquement par un ou deux composés seulement, dites « touche » ASE spécifiques, et « 20 touches » ASE déplacés dans la même direction et induits par plusieurs ou la totalité des composés testés, dites « touches » ASE non-spécifiques.

Les 20 « touches » ASE non-spécifiques ont été testés une nouvelle fois par RT-PCR au regard de 5 conditions : PBMC, PBMC + DMSO, Darunavair, drug402 et drug464.

L'analyse par électrophorèse sur gel d'agarose n'a pas confirmé de déplacement significatif à l'égard de la plupart des « touches » ASE entre l'échantillon contrôle et l'échantillon traité, à l'exception des « touches » relatives aux gènes RBM39 et CKLF (Figure 1 A).

Deux « touches » ASE concernant les gènes RBM39 et CKLF ont été testées à nouveaux pour les 12 conditions initiales (Figure 1B).

Seul le déplacement de l'ASE du gène RBM39 a été confirmé pour tous les composés testés.

Ces résultats montrent que la détermination de la transcription des isoformes longue et courte du gène RBM39, et en particulier un déplacement de l'ASE du gène RBM39, peut être mis en oeuvre comme biomarqueur de l'efficacité d'agents actifs à l'égard d'une infection du VIH par action sur le mécanisme d'épissage alternatif.

## Revendications

1. Utilisation (i) d'un taux d'expression d'une isoforme longue d'une protéine RBM39, dite RBM39L, et (ii) d'un taux d'expression d'une isoforme courte d'une protéine RBM39, dite RBM39C, comme marqueur de l'efficacité d'un actif apte à prévenir et/ou traiter une infection à VIH ;
- le rapport entre ledit taux d'expression de l'isoforme longue et ledit taux d'expression de l'isoforme courte étant indicatif de l'efficacité de l'actif à prévenir et/ou traiter la dite infection ;
- la dite isoforme longue correspondant à une isoforme de la protéine RBM39 de référence UniProtKB/Swiss-Prot Q14498-1;
- la dite isoforme courte correspondant à une isoforme de la protéine RBM39 de séquence SEQ ID N°3.

2. Utilisation selon la revendication 1, **caractérisée en ce que** lesdits taux d'expression consistent respectivement en (i) un taux de transcription d'un ARNm codant pour une isoforme longue, dite ARNmL, et (ii) un taux de transcription d'un ARNm codant pour une isoforme courte, dite ARNmC, d'une protéine RBM39.

3. Utilisation selon la revendication 1, **caractérisée en ce que** lesdits taux d'expression consistent respectivement en (i) un taux de production d'une isoforme longue, dite ProtL, et en (ii) un taux de production d'une isoforme courte, dite ProtC, d'une protéine RBM39.

4. Utilisation selon l'une des revendications précédentes, dans laquelle un rapport du taux d'expression de RBM39L à la somme des taux d'expression de RBM39L et de RBM39C est déterminé.

5. Utilisation selon l'une des revendications 1, 2 et 4, dans laquelle les taux d'expression consistent en des taux de transcription et sont déterminés par une technique de quantification avec amplification d'acides nucléiques.

6. Utilisation selon l'une des revendications 1 et 3, dans laquelle les taux d'expression consistent en des taux de production et sont déterminés par une méthode d'immunodétection.

7. Procédé de criblage in vitro ou ex vivo d'un actif présumé prévenir et/ou traiter une infection à VIH comprenant au moins les étapes consistant à :
a- mettre en contact :
i- au moins une cellule convenant à la transcription, par épissage alternatif, d'un ARNm codant pour une isoforme longue, ARNmL, et d'un ARNm codant pour une isoforme courte, ARNmC, de la protéine RBM39, ladite cellule étant disposée dans des conditions propices à ladite transcription, et
ii- un actif à cribler,
dans des conditions propices à l'observation d'un éventuel effet dudit actif sur la transcription d'un gène RBM39,
b- déterminer un taux de transcription d'un ARNmL et un taux de transcription d'un ARNmC dudit gène RBM39,
c- déterminer un rapport entre les taux de transcription déterminés à l'étape b),
d- comparer ledit rapport obtenu à l'étape c) à un rapport de référence ;
- la dite isoforme longue correspondant à une isoforme de la protéine RBM39 de référence UniProtKB/Swiss-Prot Q14498-1;
- la dite isoforme courte correspondant à une isoforme de la protéine RBM39 de séquence SEQ ID N°3.

8. Procédé selon la revendication précédente, dans lequel le rapport déterminé à l'étape c) consiste au rapport du taux de transcription de l'ARNmL à la somme des taux de transcription de l'ARNmL et de l'ARNmC.

9. Procédé selon l'une des revendications 7 ou 8, dans lequel une déviation entre le rapport obtenu à l'étape c) et le rapport de référence est indicative d'un éventuel effet dudit actif à tester.

10. Procédé selon l'une quelconque des revendications 7 à 9, dans lequel les taux de transcription des ARNmL et ARNmC codant pour une protéine RBM39 sont obtenus au moyen d'une technique de quantification avec amplification d'acides nucléiques, de préférence une méthode choisie parmi RT-PCR, et RTqPCR.

11. Procédé d'évaluation de l'efficacité d'un actif destiné à prévenir et/ou traiter une infection à VIH chez un individu en ayant besoin comprenant au moins les étapes consistant à :
a- déterminer, dans un échantillon biologique isolé dudit individu, avant traitement avec ledit actif, un taux d'expression d'une isoforme longue d'une protéine RBM39, RBM39L, et un taux d'expression d'une isoforme courte d'une protéine RBM39, RBM39C,
b- déterminer un rapport entre les taux d'expression déterminés à l'étape a),
c- déterminer, dans un échantillon biologique isolé dudit individu, après traitement avec ledit actif, un taux d'expression d'une isoforme longue d'une protéine RBM39, RBM39L, et un taux d'expression d'une isoforme courte d'une protéine RBM39, RBM39C,
d- déterminer un rapport entre les taux d'expression déterminés à l'étape c), et
e- comparer ledit rapport obtenu à l'étape d) à ledit rapport obtenu à l'étape b) ;
- la dite isoforme longue correspondant à une isoforme de la protéine RBM39 de référence UniProtKB/Swiss-Prot Q14498-1;
- la dite isoforme courte correspondant à une isoforme de la protéine RBM39 de séquence SEQ ID N°3.

12. Procédé selon la revendication 11, **caractérisé en ce que** lesdits taux d'expression consistent respectivement en (i) un taux de transcription d'un ARNm codant pour une isoforme longue, ARNmL, et (ii) un taux de transcription d'un ARNm codant pour une isoforme courte, ARNmC, d'une protéine RBM39.

13. Procédé selon la revendication 11, **caractérisé en ce que** lesdits taux d'expression consistent respectivement en (i) un taux de production d'une isoforme longue, ProtL, et en (ii) un taux de production d'une isoforme courte, ProtC, d'une protéine RBM39.

14. Utilisation d'un kit comprenant au moins une paire d'amorces d'acides nucléiques convenant à la détermination d'un taux de transcription d'un ARNm codant pour une isoforme longue, ARNmL, et un taux de transcription d'un ARNm codant pour une isoforme courte, ARNmC, d'une protéine RBM39, pour déterminer l'efficacité d'un actif apte à prévenir et/ou traiter une infection à VIH ;
- le rapport entre ledit taux d'expression de l'isoforme longue et ledit taux d'expression de l'isoforme courte étant indicatif de l'efficacité de l'actif à prévenir et/ou traiter la dite infection ;
- la dite isoforme longue correspondant à une isoforme de la protéine RBM39 de référence UniProtKB/Swiss-Prot Q14498-1;
- la dite isoforme courte correspondant à une isoforme de la protéine RBM39 de séquence SEQ ID N°3.

15. Utilisation d'un kit comprenant au moins un anticorps convenant à la détermination (i) d'un taux de production d'une isoforme longue, ProtL, et en (ii) d'un taux de production d'une isoforme courte, ProtC, d'une protéine RBM39., pour déterminer l'efficacité d'un actif apte à prévenir et/ou traiter une infection à VIH ;
- le rapport entre ledit taux d'expression de l'isoforme longue et ledit taux d'expression de l'isoforme courte étant indicatif de l'efficacité de l'actif à prévenir et/ou traiter la dite infection ;
- la dite isoforme longue correspondant à une isoforme de la protéine RBM39 de référence UniProtKB/Swiss-Prot Q14498-1;
- la dite isoforme courte correspondant à une isoforme de la protéine RBM39 de séquence SEQ ID N°3.

## Patentansprüche

1. Verwendung (i) eines Expressionsniveaus einer langen Isoform eines RBM39-Proteins, und zwar RBM39L, und (ii) eines Expressionsniveaus einer kurzen Isoform eines RBM39-Proteins, und zwar RBM39C, als Marker für die Wirksamkeit eines Wirkstoffes, der zur Vorbeugung und/oder Behandlung einer HIV-Infektion geeignet ist;
- wobei das Verhältnis zwischen dem Expressionsniveau der langen Isoform und dem Expressionsniveau der kurzen Isoform ein Hinweis ist für die Wirksamkeit des Wirkstoffs zur Vorbeugung und/oder Behandlung der Infektion;
- wobei die lange Isoform einer Isoform des RBM39-Referenzproteins UniProtKB/Swiss-Prot Q14498-1 entspricht;
- die kurze Isoform einer Isoform des RBM39-Proteins der Sequenz SEQ ID NO: 3 entspricht.

2. Verwendung nach Anspruch 1, **dadurch gekennzeichnet, dass** die Expressionsniveaus jeweils bestehen aus (i) einem Transkriptionsniveau einer mRNA, die eine lange Isoform codiert, und zwar mLRNA, und (ii) einem Transkriptionsniveau einer mRNA, die eine kurze Isoform codiert, die mCRNA genannt wird, eines RBM39-Proteins.

3. Verwendung nach Anspruch 1, **dadurch gekennzeichnet, dass** die Expressionsniveaus jeweils bestehen aus (i) einem Produktionsniveau einer langen Isoform, und zwar ProtL, und (ii) einem Produktionsniveau einer kurzen Isoform, und zwar ProtC, eines RBM39-Proteins.

4. Verwendung nach einem der vorgenannten Ansprüche, wobei ein Verhältnis des Expressionsniveaus von RBM39L zur Summe der Expressionsniveaus von RBM39L und RBM39C bestimmt wird.

5. Verwendung nach einem der Ansprüche 1, 2 und 4, wobei die Expressionsniveaus aus Transkriptionsniveaus bestehen und durch eine Quantifizierungstechnik mit Amplifikation von Nukleinsäuren bestimmt werden.

6. Verwendung nach einem der Ansprüche 1 bis 3, wobei die Expressionsniveaus aus Produktionsniveaus bestehen und durch ein Immundetektionsverfahren bestimmt werden.

7. In-vitro- oder Ex-vivo-Screeningverfahren eines Wirkstoffs, von dem die Prävention und/oder Behandlung einer HIV-Infektion angenommen wird, umfassend mindestens die folgenden Schritte:
a- In-Kontakt-Bringen von:
i- mindestens einer Zelle, die zur Transkription durch abwechselndes Spleißen einer mRNA, die eine lange Isoform mLRNA codiert, und einer mRNA, die eine kurze Isoform mCRNA codiert, des RBM39-Proteins geeignet ist, wobei die Zelle unter Bedingungen gehalten wird, die für die Transkription günstig sind, und
ii- einem zu screenenden Wirkstoff,
unter Bedingungen, die für die Ermittlung einer möglichen Wirkung des Wirkstoffs auf die Transkription eines RBM39-Gens günstig sind,
b- Bestimmen eines Transkriptionsniveaus einer mLRNA und eines Transkriptionsniveaus einer mCRNA des RBM39-Gens,
c- Bestimmen eines Verhältnisses zwischen den in Schritt b) bestimmten Transkriptionsniveaus,
d- Vergleichen des in Schritt c) erhaltenen Verhältnisses mit einem Referenzverhältnis;
- wobei die lange Isoform einer Isoform des RBM39-Referenzproteins UniProtKB/Swiss-Prot Q14498-1 entspricht;
- wobei die kurze Isoform einer Isoform des RBM39-Proteins der Sequenz der SEQ ID NO: 3 entspricht.

8. Verfahren nach dem vorangehenden Anspruch, wobei das in Schritt c) bestimmte Verhältnis aus dem Verhältnis des Transkriptionsniveaus der mLRNA zur Summe der Transkriptionsniveaus der mLRNA und der mCRNA besteht.

9. Verfahren nach einem der Ansprüche 7 oder 8, wobei eine Abweichung zwischen dem in Schritt c) erhaltenen Verhältnis und dem Referenzverhältnis ein Hinweis ist auf eine mögliche Wirkung des zu testenden Wirkstoffs.

10. Verfahren nach einem der Ansprüche 7 bis 9, wobei das Transkriptionsniveau der mLRNA und der mCRNA, die ein RBM39-Protein codieren, mittels einer Quantifizierungstechnik mit Amplifikation von Nukleinsäuren erhalten werden, vorzugsweise ein Verfahren, ausgewählt aus RT-PCR und RTqPCR.

11. Verfahren zur Bewertung der Wirksamkeit eines Wirkstoffs, der zur Vorbeugung und/oder Behandlung einer HIV-Infektion bei einem Individuum mit Bedarf daran, bestimmt ist, umfassend mindestens die folgenden Schritte:
a- Bestimmen in einer aus dem Individuum isolierten biologischen Probe, vor der Behandlung mit dem Wirkstoff, eines Expressionsniveaus einer langen Isoform eines RBM39-Proteins, RBM39L, und eines Expressionsniveaus einer kurzen Isoform eines RBM39-Proteins, RBM39C,
b- Bestimmen eines Verhältnisses zwischen den in Schritt a) bestimmten Expressionsniveaus,
c- Bestimmen, in der aus dem Individuum isolierten biologischen Probe, nach Behandlung mit dem Wirkstoff, eines Expressionsniveaus einer langen Isoform eines RBM39-Proteins, RBM39L, und eines Expressionsniveaus einer kurzen Isoform eines RBM39-Proteins, RBM39C,
d- Bestimmen eines Verhältnisses zwischen den in Schritt c) bestimmten Expressionsniveaus und
e- Vergleichen des in Schritt d) erhaltenen Verhältnisses mit dem in Schritt b) erhaltenen Verhältnis;
- wobei die lange Isoform einer Isoform des RBM39-Referenzproteins UniProtKB/Swiss-Prot Q14498-1 entspricht;
- wobei die kurze Isoform einer Isoform des RBM39-Proteins der Sequenz SEQ ID NO: 3 entspricht.

12. Verfahren nach Anspruch 11, **dadurch gekennzeichnet, dass** die Expressionsniveaus jeweils bestehen aus (i) einem Transkriptionsniveau einer mRNA, die eine lange Isoform, mLRNA, codiert, und (ii) einem Expressionsniveau einer mRNA, die eine kurze Isoform, mCRNA, codiert, eines RBM39-Proteins.

13. Verfahren nach Anspruch 11, **dadurch gekennzeichnet, dass** die Expressionsniveaus jeweils bestehen aus (i) einem Produktionsniveau einer langen Isoform ProtL und aus (ii) einem Produktionsniveau einer kurzen Isoform ProtC eines RBM39-Proteins.

14. Verwendung eines Kits, umfassend mindestens ein Paar von Nukleinsäureprimern, die zur Bestimmung eines Transkriptionsniveaus einer mRNA geeignet sind, die eine lange Isoform, mLRNA, codiert, und eines Transkriptionsniveaus einer mRNA, die eine kurze Isoform, mCRNA, codiert, eines RBM39-Proteins, zur Bestimmung der Wirksamkeit eines zur Prävention und/oder Behandlung einer HIV-Infektion geeigneten Wirkstoffs;
- wobei das Verhältnis zwischen dem Expressionsniveau der langen Isoform und dem Expressionsniveau der kurzen Isoform ein Hinweis für die Wirksamkeit des Wirkstoffs zur Prävention und/oder Behandlung der Infektion ist;
- wobei die lange Isoform einer Isoform des RBM39-Referenzproteins UniProtKB/Swiss-Prot Q14498-1 entspricht;
- die kurze Isoform einer Isoform des RBM39-Proteins der Sequenz SEQ ID NO: 3 entspricht.

15. Verwendung eines Kits, umfassend mindestens einen Antikörper, der zur Bestimmung (i) eines Produktionsniveaus einer langen Isoform, ProtL, geeignet ist, und (ii) eines Produktionsniveaus einer kurzen Isoform, ProtC, eines RBM39-Proteins zur Bestimmung der Wirksamkeit eines zur Prävention und/oder Behandlung einer HIV-Infektion geeigneten Wirkstoffs;
- wobei das Verhältnis zwischen dem Expressionsniveau der langen Isoform und dem Expressionsniveau der kurzen Isoform ein Hinweis ist für die Wirksamkeit des Wirkstoffs zur Prävention und/oder Verhinderung der Infektion;
- wobei die lange Isoform einer Isoform des RBM39-Referenzproteins UniProtKB/Swiss-Prot Q14498-1 entspricht;
- die kurze Isoform einer Isoform des RBM39-Proteins der Sequenz SEQ ID NO: 3 entspricht.

## Claims

1. Use of (i) a level of expression of a long isoform of a RBM39 protein, called RBM39L, and (ii) an expression level of a short isoform of a RBM39 protein, called RBM39S, as a marker of the efficacy of an active substance suitable for preventing and/or treating HIV infection.
- the ratio between the expression level of the long isoform and the expression level of the short isoform being indicative of the efficacy of an active substance suitable for preventing and/or treating the HIV infection;
- the long isoform corresponding to an isoform of RBM39 protein referenced UniprotKB/Swiss-Prot Q14498-1;
- the short isoform corresponding to an isoform of RMB39 protein of sequence SEQ ID N°3.

2. The use according to claim 1, **characterized in that** said expression levels consist respectively of (i) a rate of transcription of an mRNA encoding a long isoform, called mRNA-L, and (ii) a rate of transcription of an mRNA encoding a short isoform, called mRNA-S, of a RBM39 protein.

3. The use according to claim 1, **characterized in that** said expression levels consist respectively of (i) a rate of production of a long isoform, called ProtL, and (ii) a rate of production of a short isoform, called ProtS, of a RBM39 protein.

4. The use according to one of the preceding claims, wherein a ratio of the expression levels of RBM39L to the sum of the expression levels of RBM39L and of RBM39S is determined.

5. The use according to one of claims 1, 2 and 4, wherein the expression levels consist of transcription levels and are determined by a technique of quantification with amplification of nucleic acids.

6. The use according to one of claims 1 to 4, wherein the expression levels consist of levels of production and are determined by a method of immunodetection.

7. A method of screening *in vitro* or *ex vivo* of an active substance that is presumed to prevent and/or treat an HIV infection comprising at least the steps consisting of:
a- bringing in contact:
i- at least one cell suitable for the transcription, by alternative splicing, of an mRNA coding for a long isoform, mRNA-L, and of an mRNA coding for a short isoform, mRNA-S, of the RBM39 protein, said cell being disposed in conditions that are favorable to said transcription, and
ii- an active substance to be screened,
in conditions favorable to observation of any effect of said active substance on the transcription of an RBM39 gene,
b- determining a transcription level of an mRNA-L and a transcription level of an mRNA-S of said RBM39 gene,
c- determining a ratio of the transcription levels determined in step b),
d- comparing said ratio obtained in step c) with a reference ratio;
- the long isoform corresponding to an isoform of RBM39 protein referenced UniprotKB/Swiss-Prot Q14498-1;
- the short isoform corresponding to an isoform of RMB39 protein of sequence SEQ ID N°3.

8. The method as claimed in the preceding claim, wherein the ratio determined in step c) consists of the ratio of the transcription level of mRNA-L to the sum of the transcription levels of mRNA-L and mRNA-S.

9. The method as claimed in claim 7 or 8, wherein a deviation between the ratio obtained in step c) and the reference ratio is indicative of a possible effect of said active substance to be tested.

10. The method as claimed in any one of claims 7 to 9, wherein the transcription levels of mRNA-L and mRNA-S coding for an RBM39 protein are obtained by means of a technique of quantification with amplification of nucleic acids, preferably a method selected from RT-PCR, and RTqPCR.

11. A method of evaluating the efficacy of an active substance intended for preventing and/or treating HIV infection in an individual in need thereof comprising at least the steps of:
a- determining, in an isolated biological sample from said individual before treatment with said active agent, a level of expression of a long isoform of a RBM39 protein, RBM39L, and an expression level of a short isoform RBM39 protein, RBM39S,
b- determining a ratio between the expression levels determined in step a),
c- determining, in an isolated biological sample from said individual after treatment with said active substance, a level of expression of a long isoform protein RBM39, RBM39L, and an expression level of a short isoform of a protein RBM39, RBM39S,
d- determining a ratio between the expression level determined in step c) and
e- comparing said ratio obtained in step d) with said ratio obtained in step b) ;
- the long isoform corresponding to an isoform of RBM39 protein referenced UniprotKB/Swiss-Prot Q14498-1;
- the short isoform corresponding to an isoform of RMB39 protein of sequence SEQ ID N°3.

12. The method according to claim 11, **characterized in that** said expression levels consist respectively of (i) rate of transcription of an mRNA encoding a long isoform, mRNA-L, and (ii) a rate of transcription of a mRNA coding for a short isoform, mRNA-S, of a RBM39 protein.

13. The method according to claim 11, **characterized in that** said expression levels consist respectively of (i) a rate of production of a long isoform, ProtL, and (ii) a rate of production of a short isoform, ProtS, of a RBM39 protein.

14. Use of a kit comprising at least one pair of nucleic acid primers suitable for determining a rate of transcription of an mRNA encoding a long isoform, mRNA-L, and rate of transcription of an mRNA encoding a short isoform, mRNA-S, of a RBM39 protein, for determining the efficacy of an active substance for preventing and/or treating an HIV infection;
- the ratio between the expression level of the long isoform and the expression level of the short isoform being indicative of the efficacy of an active substance suitable for preventing and/or treating the HIV infection;
- the long isoform corresponding to an isoform of RBM39 protein referenced UniprotKB/Swiss-Prot Q14498-1;
- the short isoform corresponding to an isoform of RMB39 protein of sequence SEQ ID N°3.

15. Use of a kit comprising at least one antibody suitable for determining (i) a rate of production of a long isoform, ProtL, and (ii) a rate of production of a short isoform, ProtS, of a RBM39 protein, for determining the efficacy of an active substance for preventing and/or treating an HIV infection
- the ratio between the expression level of the long isoform and the expression level of the short isoform being indicative of the efficacy of an active substance suitable for preventing and/or treating the HIV infection;
- the long isoform corresponding to an isoform of RBM39 protein referenced UniprotKB/Swiss-Prot Q14498-1;
- the short isoform corresponding to an isoform of RMB39 protein of sequence SEQ ID N°3.
